(19) Europäisches Patentamt
European Patent Office
Office européen des brevets

(11) **EP 2 763 068 A2**

(12) **EUROPEAN PATENT APPLICATION**

(43) Date of publication:
06.08.2014 Bulletin 2014/32

(51) Int Cl.:
*G06F 19/12* (2011.01)

(21) Application number: 13198594.7

(22) Date of filing: 19.12.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 31.01.2013 JP 2013017681

(71) Applicants:
• FUJITSU LIMITED
Kawasaki-shi,
Kanagawa 211-8588 (JP)
• The University of Tokyo
Bunkyo-ku,
Tokyo 113-8654 (JP)

(72) Inventors:
• Washio, Takumi
Bunkyo-ku, Tokyo 113-8654 (JP)

• Okada, Jun-ichi
Bunkyo-ku, Tokyo 113-8654 (JP)
• Takahashi, Akihito
Bunkyo-ku, Tokyo 113-8654 (JP)
• Sugiura, Seiryo
Bunkyo-ku, Tokyo 113-8654 (JP)
• Hisada, Toshiaki
Bunkyo-ku, Tokyo 113-8654 (JP)
• Yoneda, Kazunori
Kawasaki-shi, Kanagawa 211-8588 (JP)
• Matsunaga, Hiroyuki
Fukuoka-shi, Fukuoka 814-8589 (JP)

(74) Representative: Schultes, Stephan
Haseltine Lake LLP
300 High Holborn
London WC1V 7JH (GB)

(54) **Biological simulation program, biological simulation method, and biological simulation device**

(57) A biological simulation method of causing a computer to execute following steps. Firstly, the computer calculates states of a plurality of actins and states of a plurality of myosins in a sarcomere contained in a muscle of a biological body using a model that defines a plurality of states of the actins and a plurality of states of the myosins and transition rates between the states. Secondly, the computer calculates behaviors of the respective actins and behaviors of the respective myosins based on the states of the actins and the states of the myosins, respectively. Thirdly, the computer calculates a behavior of the sarcomere based on the behaviors of the actins and the behaviors of the myosins. Fourthly, the computer calculates a behavior of the muscle based on the behavior of the sarcomere.

FIG.1

**Description**

FIELD

The embodiments discussed herein are related to a biological simulation program, a biological simulation method, and a biological simulation device.

BACKGROUND

In the field of molecular biology, diverse sarcomere models have been proposed that describe cross-bridge interactions between myosins and actins in sarcomeres based on experimental facts. One exemplary sarcomere model defines a plurality of representative states of molecules contributing to the binding between the myosin and the actin in the sarcomere and defines the transition rate between these states in consideration of interactions among the neighboring molecules, energy of the molecules, and the like. Generally, in a coupling analysis of the molecular motions in sarcomeres and the muscular continuum model, an ordinary differential equation is derived where a state concentration is set as a variable based on the average behavior of the molecular models. Usually, a contraction force on the continuum model is calculated based on calculation results of the sarcomere models and motion of muscle as the continuum is calculated based on the result.

Various researches have been made as can be seen in: Peterson J.N., Hunter W.C., Berman M.R.: Estimated time course of Ca2+bound to troponin C during relaxation in isolated cardiac muscle, American Physiological Society, H1013-H1024(1991); Hunter P.J., McCulloch A.D., ter Keurs H.E.D.J.: Modeling the mechanical properties of cardiac muscle, Progress in Biophysics & Molecular Biology 69, 289-331(1998); Razumova M.V., Bukatina A.E., Campbell K.B.: Stiffness-distortion sarcomere model for muscle simulation. J. Appl. Physiol. 87, 1861-1876(1999); Rice J.J., Wang F., Bers D.M., de Tombe P.P.: Approximate model of cooperative activation and crossbridge cycling in cardiac muscle using ordinary differential equations. Biophys. J. 95, 2368-2390(2008).

The above-mentioned sarcomere models indicate an averaged behavior of the molecular models in the sarcomere models. The simulation of behaviors of sarcomeres using these sarcomere models, therefore, provides simulation results lacking accuracy with respect to the state transitions of the molecular models. This results in a problem that a simulation result for motion of a muscle as the continuum also lacks accuracy. Furthermore, the muscular motion is strongly fed back to the state transitions of the molecules in the sarcomere model. For this reason, it is difficult to execute a reliable analysis by the simulation based on one-way information transmission from the sarcomere models to the continuum model.

Accordingly, it is an object in one aspect of an embodiment of the invention to provide an accurate result of a coupling simulation of molecular models having stochastic behaviors and a motion of muscle as a continuum. It is an object in another aspect of an embodiment of the invention to perform a coupling analysis stably in an appropriate time step through taking a feedback of the motion of the muscular continuum model to the molecular models into the coupling simulation based on an implicit solution.

SUMMARY

According to an aspect of an embodiment, a biological simulation method and a biological simulation program causes a computer to execute a following process. Firstly, calculating states of a plurality of actins and states of a plurality of myosins in a sarcomere contained in a muscle of a biological body using a model that defines a plurality of states of the actins and a plurality of states of the myosins and transition rates between the states. Secondly, calculating behaviors of the respective actins and behaviors of the respective myosins based on the states of the actins and the states of the myosins, respectively. Thirdly, calculating a behavior of the sarcomere based on the behaviors of the actins and the behaviors of the myosins. Fourthly, calculating a behavior of the muscle based on the behavior of the sarcomere.

According to another aspect of an embodiment, a biological simulation device includes a first calculator, a second calculator, and a third calculator. The first calculator is configured to calculate states of a plurality of actins and states of a plurality of myosins in a sarcomere contained in a muscle of a biological body using a model that defines a plurality of states of the actins and a plurality of states of the myosins and transition rates between the states. The second calculator is configured to calculate behaviors of the respective actins and behaviors of the respective myosins based on the states of the actins and the states of the myosins, respectively. The third calculator is configured to calculate a behavior of the sarcomere based on the behaviors of the actins and the behaviors of the myosins and calculate a behavior of the muscle based on the behavior of the sarcomere.

BRIEF DESCRIPTION OF DRAWINGS

FIG. 1 is a diagram illustrating an example of a functional configuration of a biological simulation device according to an embodiment;

FIG. 2 is a view illustrating a part of an example of a myocardial model;

FIG. 3 is a view illustrating an example of a sarcomere model;

FIG. 4 is a view illustrating an example of a state transition of a T/T unit;

FIG. 5 is a view illustrating an example of a state transition of a myosin head;

FIG. 6 is a view for explaining a change of an overlap state of actin filaments in accordance with sarcomere length (SL);

FIG. 7 is a flowchart illustrating procedures of biological simulation processing in the embodiment;

FIG. 8 is a flowchart illustrating procedures of simulation processing in the embodiment;

FIG. 9 is a view for explaining an example of a calculation method of a stretch of a myosin arm;

FIG. 10 is a flowchart illustrating procedures of Monte Carlo simulation processing in the embodiment;

FIG. 11 is a flowchart illustrating procedures of a finite element analysis in the embodiment;

FIG. 12 is a view for explaining transition information of the T/T unit that is received by a receiver in the embodiment;

FIG. 13 is a view for explaining information of the myosin head that is received by the receiver in the embodiment;

FIG. 14 is a view for explaining function information that relates to a state of the T/T unit and is received by the receiver in the embodiment;

FIG. 15 is a view for explaining function information that relates to a state of the myosin head and is received by the receiver in the embodiment;

FIG. 16 is a view for explaining information that defines a transition between a non-binding state and a binding state of the myosin head and is received by the receiver in the embodiment;

FIG. 17 is a view for explaining information that defines a transition between states before and after swing of the myosin head and is received by the receiver in the embodiment;

FIG. 18 is a view for explaining information that defines transition between binding and dissociation that is received by the receiver in the embodiment;

FIG. 19 is a view illustrating a result of automatic generation of a code (Monte Carlo code) for executing a Monte Carlo step based on the definition of states of the myosin head and state transitions;

FIG. 20A is a view illustrating an example of parameter specification relating to a myocardial continuum model when simulation of heart beat is executed;

FIG. 20B is a view illustrating an example of a method of specifying the fiber direction when the simulation of the heart beat is executed;

FIG. 21A is a table illustrating an example of a simulation result of various amounts relating to performance of the heart beat;

FIG. 21B is a graph illustrating an example of simulation results of left ventricle pressure-volume change (upper graph) and work rate and energy consumption rate (lower graph);

FIG. 21C is a view illustrating an example of a simulation result relating to distribution of a contraction force; and

FIG. 22 is a diagram illustrating a computer that executes a biological simulation program.

DESCRIPTION OF EMBODIMENTS

Preferred embodiments of the present invention will be explained with reference to accompanying drawings. Note that the embodiments do not limit disclosed techniques.

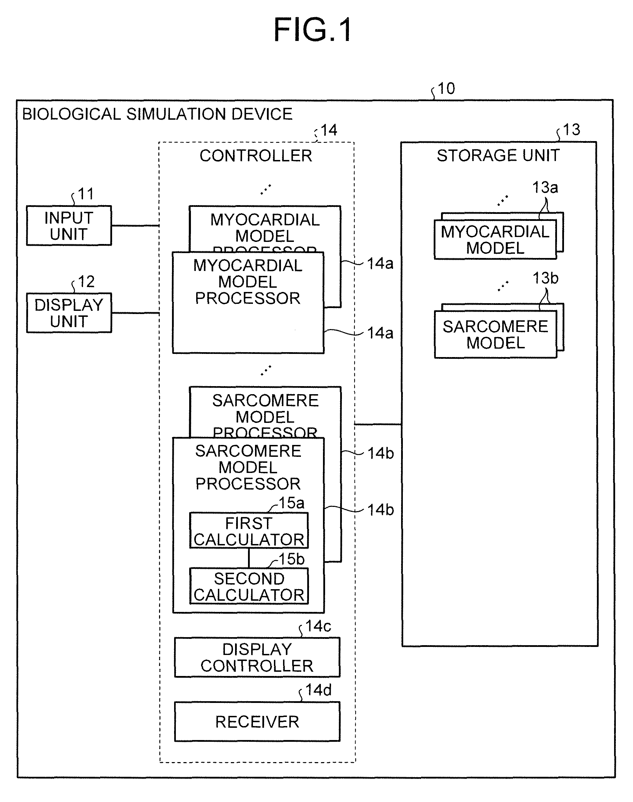

The following describes the biological simulation device according to an embodiment. The biological simulation device in the embodiment performs the following processing using a model that defines a plurality of states of a plurality of actins and a plurality of myosins in a sarcomere contained in a muscle of a biological body and transition rates between the states, and a muscular continuum model expressed in a finite element mesh. That is to say, the biological simulation device calculates state transitions of the actins and the myosins that are embedded in finite elements of the muscular continuum model. The biological simulation device solves an equation of motion with which contraction forces generated by respective molecules, which are derived from state transitions of the molecular models and motion of the muscle as a continuum, are matched with a contraction force of the continuum model of the finite elements into which molecular models are embedded to perform the following analysis. That is, the biological simulation device performs an analysis that couples the molecular state transition motion and the muscular motion. FIG. 1 is a diagram illustrating a functional configuration of the biological simulation device according to the embodiment. As illustrated in FIG. 1, this biological simulation device 10 includes an input unit 11, a display unit 12, a storage unit 13, and a controller 14.

The input unit 11 inputs various kinds of information to the controller 14. For example, upon receiving an instruction to execute biological simulation processing, which will be described later, from a user, the input unit 11 inputs the received instruction to the controller 14. Examples of a device as the input unit 11 includes a keyboard and a mouse.

The display unit 12 displays various kinds of information. For example, the display unit 12 displays a simulation result under control by a display controller 14c, which will be described later.

The storage unit 13 stores therein various types of programs that are executed by the controller 14. The storage unit 13 stores therein myocardial models 13a and sarcomere models 13b.

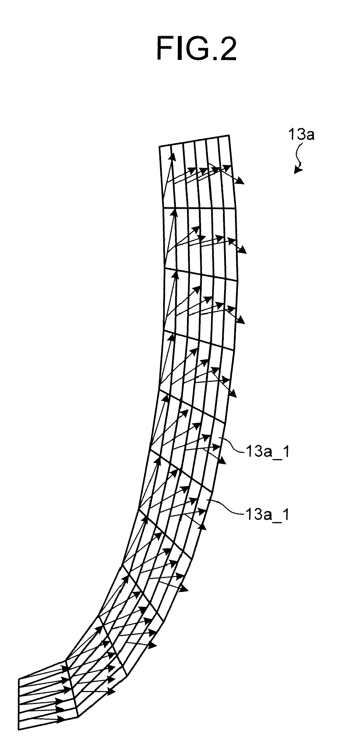

The myocardial models 13a are models obtained by dividing a muscular model of the entire heart as a continuum into a plurality of elements. For example, the muscular model of the entire heart is divided into four models of the left atrium, the left ventricle, the right atrium, and the right ventricle, and each of the left atrium model, the left ventricle model, the right atrium model, and the right ventricle model can be set as the myocardial model 13a. The myocardial model 13a is used to calculate a behavior of a muscular model indicated by the myocardial model 13a in the finite element analysis. FIG. 2 is a view illustrating a part of an example of the myocardial model. In the example of FIG. 2, the myocardial model 13a is the left ventricle model, and a part of the left ventricle model is illustrated. The example of FIG. 2 illustrates the fiber directions of a plurality of elements 13a_1 in the finite element mesh of the myocardial model 13a with arrows. In the following description, a vector of the fiber direction is expressed with f in some cases.

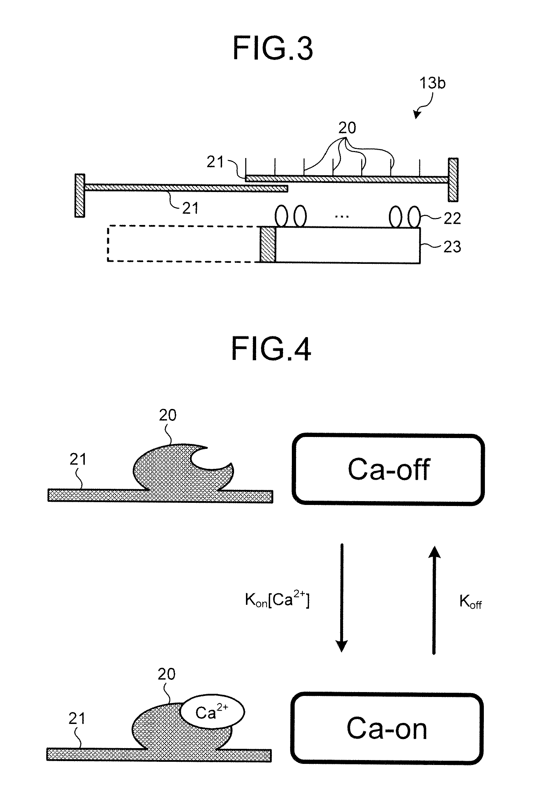

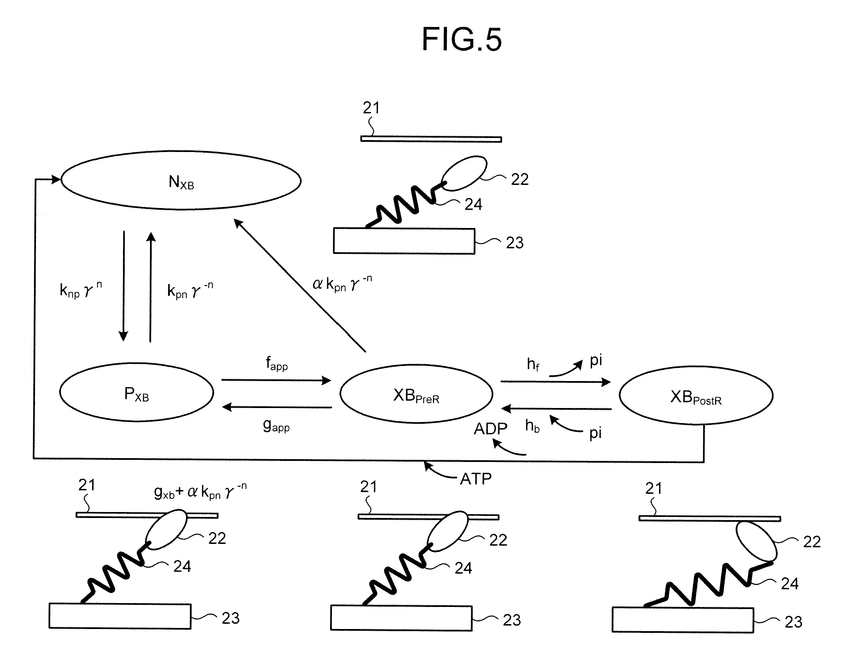

Each of the elements 13a_1 includes a plurality of sarcomere models 13b embedded therein. The following describes the case where the element 13a_1 includes "ns" sarcomere models 13b embedded therein. Each of the sarcomere models 13b is a model in which molecules as components indicate stochastic behaviors. The sarcomere model 13b is defined so as to have so-called cooperativity and so-called sarcomere length dependency. FIG. 3 is a view illustrating an example of the sarcomere model. As illustrated in FIG. 3, the sarcomere model 13b includes a plurality of T/T units (troponin/tropomyosin units) 20 on an actin filament 21 and a plurality of myosin heads 22 on a myosin filament 23. As will be described later, the sarcomere model 13b also includes myosin arms 24 connecting the myosin filament 23 and the myosin heads 22 (see FIG. 5). The following describes the case where one sarcomere model 13b includes "nm" pairs of the myosin heads 22 and the myosin arms 24.

The sarcomere model 13b defines a plurality of states for the T/T unit 20 and the myosin head 22, and Monte Carlo simulation is executed in accordance with the previously defined transition rates among the states. That is to say, the stochastic behaviors of the T/T unit 20 and the myosin head 22 are calculated through the Monte Carlo simulation.

The state transition rates of the T/T unit 20 are controlled in accordance with the states of the myosin head 22 just under the T/T unit 20. FIG. 4 is a view illustrating an example of a state transition of the T/T unit. As illustrated in FIG. 4, the embodiment includes two types of states including a state (binding state: Ca-on) where calcium ion ($Ca2^+$) binds to the T/T unit 20 and a state (non-binding state: Ca-off) where no calcium ion ($Ca^{2+}$) binds to the T/T unit 20. In the state transition illustrated in FIG. 4, for example, the state is more likely to transition to the binding state as the concentration of the calcium ion is higher. Furthermore, a dissociation probability $K_{off}$ of the calcium ion is defined in accordance with the state of the myosin head 22 just under the T/T unit 20. Furthermore, values of $K_{on}$ and $K_{off}$ change depending on whether the myosin head 22 binds to the actin section under the corresponding T/T unit 20. For example, $K_{off}$ has a small value when the myosin head 22 binds to the actin section under the T/T unit 20, and the calcium ion is less likely to dissociate from the T/T unit 20. That is to say, the T/T unit 20 in the sarcomere model 13b in the embodiment is a model having cooperativity in which the calcium ion is less likely to dissociate from the T/T unit 20 when the myosin head 22 binds to the actin section under the T/T unit 20. The binding of the calcium ion binds to the T/T unit 20 shifts the position of tropomyosin, which is a string-like protein hiding a binding site to the myosin head 22, and this facilitates the binding of the myosin head 22 to the actin section under the T/T unit 20. Thus, the binding of the calcium ion to the T/T unit 20 increases the probability that the myosin head 22 binds to the actin section under the T/T unit 20. The binding of the myosin head 22 to the actin section under the T/T unit 20 further shifts the position of the tropomyosin, and this increases the probability that a neighbor myosin head 22 neighboring the myosin binds to the actin section just above the myosin head 22. The actin section under the T/T unit 20 indicates a section partitioned by lines indicated by the reference numeral 20 in FIG. 3, and the neighbor myosin head 22 indicates a myosin head 22 in a particular range from one myosin head 22.

The state transition of the myosin head 22 is controlled in accordance with states of the neighbor myosin head 22 and the T/T unit 20 on the actin section just above the myosin head 22. FIG. 5 illustrates an example of the state transition of the myosin head. As illustrated in FIG. 5, the embodiment includes four kinds of states of $N_{XB}$, $P_{XB}$, $XB_{PreR,}$ and $XB_{PostR}$. $N_{XB}$ is a state (non-binding state) where the myosin head 22 does not bind to the actin section under the T/T unit 20. $P_{XB}$ is a state (weak-binding state) where the myosin head 22 starts binding to the actin section under the T/T unit 20. $XB_{PreR}$ is a state (strong-binding state (1)) where the chemical state of the myosin head 22 changes from that in $P_{XB}$ and the myosin head 22 binds to the actin section under the T/T unit 20 more strongly. $XB_{PostR}$ is a state (strong-binding state (2)) where the myosin head 22 keeps binding to the actin section controlled by the T/T unit 20 and a rotating motion (swing motion) of the myosin head 22 is generated from $XB_{PreR}$. In FIG. 5, $k_{np}$ is a coefficient in the transition from $N_{XB}$ to $P_{XB}$. As an overlap of the actin filaments 21 in the sarcomere model 13b is larger, a range of the actin filament to which the myosin heads 22 can bind is shorter. That is, as the overlap of the actin filaments 21 in the sarcomere model 13b is larger, the myosin heads 22 are less likely to bind to the actin. In the embodiment, the value of $k_{np}$ is smaller as the overlap of the actin filaments 21 in the sarcomere model 13b is larger, which decreases the probability that the myosin heads 22 bind to the actin.

$k_{pn}$ is a coefficient in the transition from $P_{XB}$, $XB_{PreR}$, or $XB_{PostR}$ to $N_{XB}$. In addition, $\gamma^n$ and $\gamma^{-n}$ indicate cooperativity relating to the transition between the non-binding state and the binding state of the myosin head 22. The numeral n indicates the number (0 to 2) of neighboring myosin heads 22 that bind to the actin. For example, in the case of $\gamma=80$ and the number of the neighboring myosin heads 22 that bind to the actin is "1", $\gamma^n$ is "80". That is to say, in the case illustrated in FIG. 5, the myosin head 22 is 80 times more likely to bind to the actin. In the case of $\gamma=80$ and the number of the neighboring myosin heads 22 that bind to the actin is "2", $\gamma^n$ is "6400". That is to say, in the case illustrated in FIG. 5, the myosin head 22 is 6400 times more likely to bind to the actin. In the case of $\gamma=80$ and the number of the neighboring myosin heads 22 that bind to the actin is "1", $\gamma^{-n}$ is "1/80". That is to say, in the case illustrated in FIG. 5, the myosin head 22 is 1/80 times more likely to dissociate from the actin. In the case of $\gamma=80$ and the number of the neighboring myosin heads 22 that bind to the actin is "2", $\gamma^{-n}$ is 1/6400. That is to say, in the case illustrated in FIG. 5, the myosin head 22 is 1/6400 times more likely to dissociate from the actin. Thus, the myosin head 22 that does not bind to the actin is more likely to bind to the actin when the neighboring myosin head 22 binds to the actin. Furthermore, the myosin head 22 that binds to the actin is less likely to dissociate from the actin when the neighboring myosin head 22 also binds to the actin.

In FIG. 5, adenosine triphosphate (ATP) binds to the myosin head 22 in the state of $N_{XB}$, $P_{XB}$, or $XB_{PreR}$. A hydrolysis reaction from ATP to adenosine diphosphate (ADP) + phosphoric acid (pi) generates energy.

In FIG. 5, $f_{app}$ is defined such that the state distribution is an equilibrium state based on the Boltzmann distribution law through the transition from $P_{XB}$ to $XB_{PreR}$. In addition, $g_{app}$ is defined such that the state distribution is an equilibrium state based on the Boltzmann distribution law through the transition from $XB_{PreR}$ to $P_{XB}$.

In the transition from $XB_{PreR}$ to $XB_{PostR}$ and the transition from $XB_{PostR}$ to $XB_{PreR}$, the myosin head 22 performs swing motion. In FIG. 5, $h_f$ and $h_b$ are defined such that the state distribution is an equilibrium state, based on the Boltzmann distribution law, that is defined from a sum of a chemical energy in the myosin head and the elastic energy of the myosin arm through a change of the length of the myosin arm with the swing motion of the myosin head 22. Furthermore, $g_{xb}$ in FIG. 5 is a transition rate that indicates ease of dissociation from $XB_{PostR}$ other than the above-mentioned effects of the cooperativity. In FIG. 5, $\alpha$ is a coefficient of equal to or lower than 1 that indicates resistance to dissociation of the myosin head 22 from the actin in the strong-binding states $XB_{PreR}$ and $XB_{PostR}$.

The following describes a change of the overlap state of the actin filaments 21 in accordance with the sarcomere length (SL). First, the SL can be calculated through the following method. That is to say, a stretch along the fiber direction $\lambda(T)$ is calculated for each of the elements 13a_1 in the myocardial model 13a at time T, using the following equation (1).

$$\lambda(T) \;=\; \left\| F(T)f \right\| \tag{1}$$

Note that F(T) is a deformation gradient tensor of the myocardial model 13a at the time T.

Subsequently, the velocity (stretch velocity) along the fiber direction

$$\dot{\lambda}(T)$$

is calculated for each of the elements 13a_1, using the following equation (2).

$$\dot{\lambda}(T) \;=\; \frac{1}{\left\| F(T)f \right\|} \left( \dot{F}(T)f, \; F(T)f \right) \tag{2}$$

The sarcomere length SL(T) of the sarcomere model 13b at the time T is calculated, using the following equation (3).

$$SL(T) \;=\; SL_0 \lambda(T) \tag{3}$$

Note that $SL_0$ is a sarcomere length in a no-load state when the time T is 0.

Subsequently, a shortening velocity

$$\dot{u}(T)$$

of the sarcomere model 13b is calculated, using the following equation (4).

$$\dot{u}(T) = -\frac{SL_0}{2}\dot{\lambda}(T) \qquad (4)$$

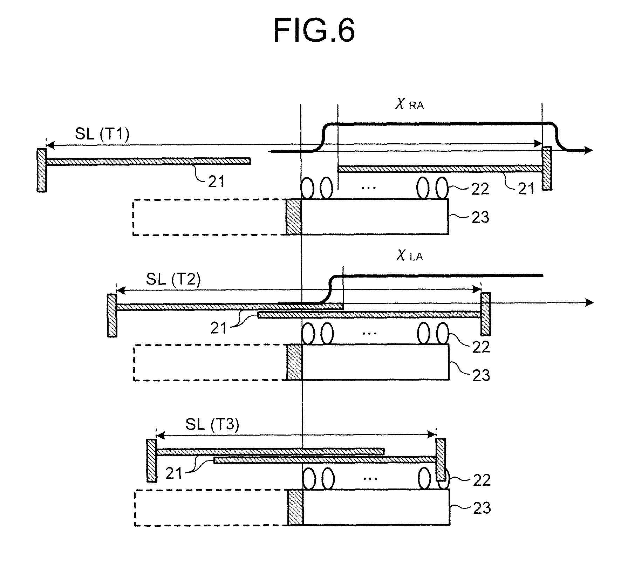

The following describes a change of the overlap state of the actin filaments 21 in accordance with the sarcomere length (SL) with reference to FIG. 6. FIG. 6 is a view for explaining the change of the overlap state of the actin filaments 21 in accordance with the SL. The example of FIG. 6 illustrates sarcomere lengths SL(T1), SL(T2), and SL(T3) at times T1, T2, and T3, respectively. As illustrated in the upper example in FIG. 6, when the sarcomere length (SL) is too large, there is no actin to which a myosin head at the center region can bind. As illustrated in the lower example in FIG. 6, when the sarcomere length (SL) is too small, an overlapping portion of the actin filaments 21 is formed in the vicinity of the center, and it is difficult for a myosin head located in this portion to bind to the actin. In consideration of the above, in the embodiment, functions $\chi_{LA}$ and $\chi_{RB}$ can be configured for reflecting the overlap state of the actin filaments to the state transition of the myosin head 22, and transition rates in the respective state transitions of the myosin head 22 to the binding state can be determined with reference to the functions $\chi_{LA}$ and $\chi_{RB}$.

In the embodiment, unlike the conventional model using the ordinary differential equation, an average behavior is not described using one representative unit. The state transitions of the respective T/T units 20 and the respective myosin heads 22 are controlled also with reference to the neighbor states. For this reason, according to the embodiment, the state transitions of the micro models are simulated in a manner closer to the reality while preventing errors due to averaging or the like. This makes it possible to perform an accurate coupling analysis with the myocardial continuum model, as will be described below.

The storage unit 13 is a storage device exemplified by a semiconductor memory element such as a flash memory, a hard disk, and an optical disk. Note that the storage unit 13 is not limited to the above-mentioned kinds of storage devices and may be a random access memory (RAM), a read only memory (ROM), or the like.

The controller 14 includes an internal memory for storing programs defining various processing procedures and control data, and executes various kinds of processing by the use of them. As illustrated in FIG. 1, the controller 14 includes a plurality of myocardial model processors 14a, a plurality of sarcomere model processors 14b, a display controller 14c, and a receiver 14d.

The myocardial model processors 14a correspond to the respective elements in the myocardial model 13a, and each of the myocardial model processors 14a calculates the behavior of the corresponding myocardial model 13a. The sarcomere model processors 14b correspond to the respective sarcomere models 13b, and each of the sarcomere model processors 14b calculates the behavior of the corresponding sarcomere model 13b. The controller 14 includes one or a plurality of multi-core central processing unit(s) (CPU(s)) having a plurality of cores as operation processors. Alternatively, the controller 14 may include a plurality of single-core CPUs having one core as the operation processor. The cores each execute a biological simulation program described later, thereby implementing each part of the myocardial model processors 14a, the sarcomere model processors 14b, the display controller 14c, and the receiver 14d.

Each of the sarcomere model processors 14b includes a first calculator 15a and a second calculator 15b.

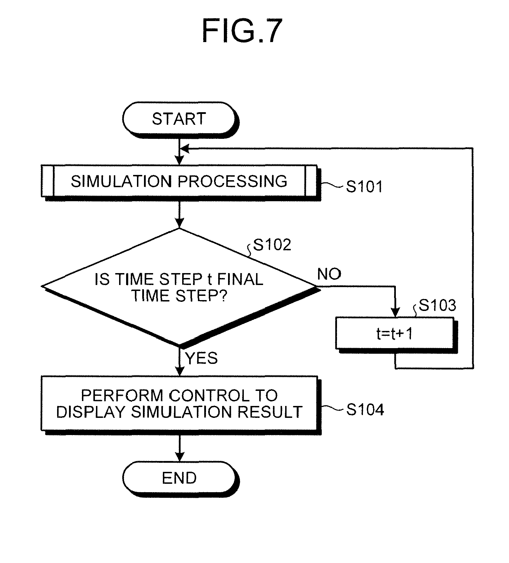

The following describes an example of processing that is executed by the controller 14 with reference to FIG. 7. FIG. 7 is a flowchart illustrating procedures of the biological simulation processing in the embodiment. The biological simulation processing is executed when the input unit 11 inputs an instruction to execute the biological simulation processing to the controller 14, for example. In the biological simulation processing, the finite element analysis of the myocardial models 13a is executed in time intervals ΔT. Furthermore, in the Monte Carlo simulation processing on the sarcomere models, simulation on the sarcomere models 13b with the Monte Carlo method is executed at time steps Δt (ΔT / n) obtained by dividing the time interval [T, T+ΔT] into small time segments of n steps. As the value of ΔT, 2.5 milliseconds can be employed, for example. Alternatively, as the value of Δt, 5 microseconds can be employed, for example. In the Monte Carlo method, a product of a transition rate and the time segment Δt is required not to be larger than 1, so that the above-mentioned small time segments is set. Although it is preferable that the calculation in the finite element analysis be also performed at small time segments in a viewpoint of the accuracy, the above-mentioned time segment ΔT is appropriate therefore because it takes time to solve the linear equation through the implicit method.

As illustrated in FIG. 7, the myocardial model processor 14a and the sarcomere model processor 14b execute the simulation processing (S101). After the simulation processing (S101), the controller 14 determines whether the time step t in which the simulation processing is performed is the final time step or not (S102). When the controller 14 determines that the time step t is not the final time step, the controller 14 adds one to t (S103) and the process returns to step S101. On the other hand, when the controller 14 determines that the time step t is the final time step, the display controller 14c performs control to display simulation result on the display unit 12 (S104).

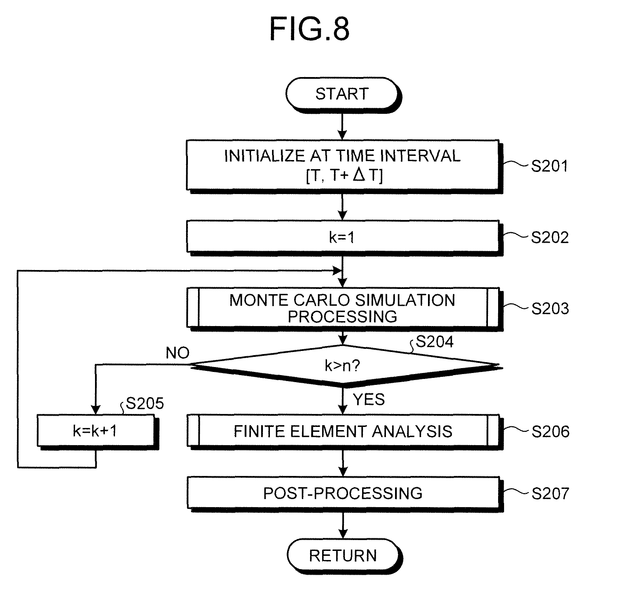

FIG. 8 is a flowchart illustrating the procedures of the simulation processing in the embodiment. As illustrated in FIG. 8, the first calculator 15a performs an initialization in the time interval [T, T+$\Delta$T] (S201). For example, the first calculator 15a sets "0" to a variable $SumL_{IR}$ at S201. The first calculator 15a sets "0" to a variable $X_{BD}$ at S201. The first calculator 15a sets "0" to a variable $X_{BD0}$ at S201. The first calculator 15a sets "1" to a flag $flag_{D0}$ at S201. The first calculator 15a sets "$L_s$ (T)" to a variable $L_{S0}$ at S201. Note that $L_s$ (T) is obtained by extracting only a content contributed by a shortening velocity between the filaments from the stretch of the myosin arm 24 at the time T. The variable is set to "0" at the processing start time and is updated to an appropriate value after every finite element step is finished, as will be described later.

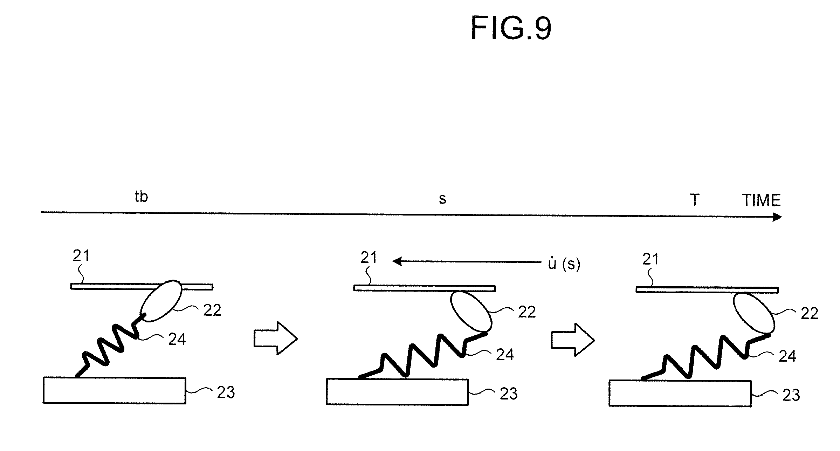

The following describes an example of a calculation method of the stretch of the myosin arm 24 with reference to FIG. 9. FIG. 9 is a view for explaining the example of the calculation method of the stretch of the myosin arm. The example of FIG. 9 illustrates the case where the myosin head 22 binds to the actin filament 21 at time tb and the binding is maintained to time T. Furthermore, the example of FIG. 9 illustrates the case where the myosin head 22 performs swing motion in the time period from the time tb to the time T. In the case of the example of FIG. 9, the first calculator 15a calculates $L_s$ (T) using the following equation (5).

$$L(T) \;=\; L_{INIT} \;+\; L_{ROT} \;-\; \int_{tb}^{T} \dot{u}(s)ds \qquad (5)$$

Note that $L_{INIT}$ is an initial stretch of the myosin arm 24 at the time of binding. $L_{ROT}$ is a stretch due to swing motion of the myosin head 22 after the binding.

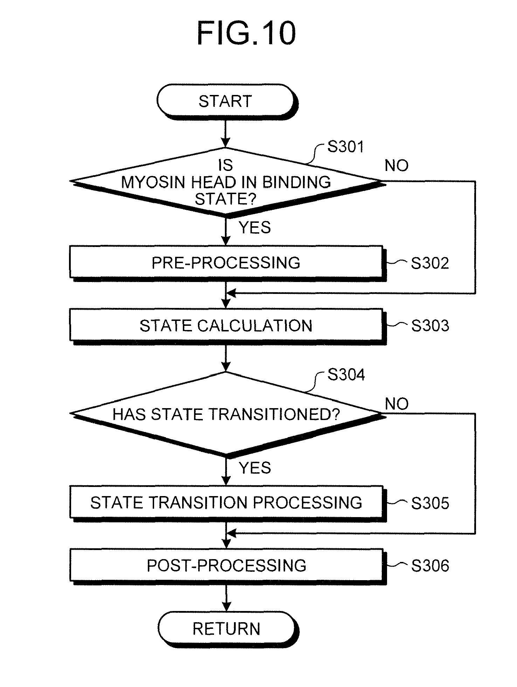

In FIG. 8, the first calculator 15a sets a value of a variable k to "1" (S202). Subsequently, the first calculator 15a and the second calculator 15b execute the Monte Carlo simulation processing (S203). The Monte Carlo simulation processing that is executed at S203 is processing at time (T+k$\Delta$t). FIG. 10 is a flowchart illustrating the procedures of the Monte Carlo simulation processing in the embodiment. As illustrated in FIG. 10, the first calculator 15a determines whether the state of the myosin head 22 is the binding state (S301). For example, at S301, when the state of the myosin head 22 is any one of $P_{XB}$, $XB_{PreR}$, and $XB_{PostR}$, the first calculator 15a determines that the myosin head 22 is in the binding state. When the state of the myosin head 22 is $N_{XB}$, the first calculator 15a determines that the myosin head 22 is not in the binding state.

When the myosin head 22 is not in the binding state (No at S301), the first calculator 15a proceeds to S303. When the myosin head 22 is in the binding state (Yes at S301), the first calculator 15a performs various kinds of pre-processing (S302). For example, the first calculator 15a increments the value of the variable $X_{BD}$ by 1 at S302. When the value of the flag $flag_{D0}$ is "1", the first calculator 15a increments the value of the variable $X_{BD0}$ by 1 at S302. In addition, the first calculator 15a calculates the stretch of the myosin arm L at S302, using the following equation (6).

$$L \;:=\; L_{IR} \;+\; L_{S0} \;-\; \Delta t XB_D \dot{u}(T) \qquad (6)$$

Note that $L_{IR}$ indicates a sum of the above-mentioned $L_{INIT}$ and $L_{ROT}$. $L_{INIT}$ is a stretch of the myosin arm immediately after the transition to the binding state, and is calculated based on the Boltzmann distribution defined from the elastic energy of the myosin arm in consideration of thermal fluctuation, for example.

The first calculator 15a generates a random number and calculates the state of the myosin head 22 using the generated random number (S303). This enables the first calculator 15a to calculate a stochastic behavior of the myosin head 22.

Thereafter, the second calculator 15b determines whether the state of the myosin head 22 has transitioned (S304). When the state of the myosin head 22 has not transitioned (No at S304), the second calculator 15b proceeds to S306.

When the state of the myosin head 22 has transitioned (Yes at S304), the second calculator 15b performs state transition processing (S305). For example, when the state of the myosin head 22 has transitioned from the non-binding state $N_{XB}$ to the binding state $P_{XB}$, the second calculator 15b sets the value of the variable $L_{INIT}$ to the variable $L_{IR}$ and sets the value of the flag $flag_{D0}$ to "0" at S305. When the state of the myosin head 22 has transitioned from any one of the binding states $P_{XB}$, $XB_{PreR}$, and $XB_{PostR}$ to the non-binding state $N_{XB}$, the second calculator 15b sets each value of the variable $L_{IR}$, the variable $L_{S0}$, and the variable $X_{Bd}$ to "0" at S305. When the myosin head 22 remains in any one of the binding states $P_{XB}$, $XB_{PreR}$, and $XB_{PostR}$ and performs the swing motion to rotate, the second calculator 15b performs the following processing at S305. Specifically, the second calculator 15b sets a sum ($L_{IR}+X_{SWING}$) of the value of the variable $L_{IR}$ and a length $X_{SWING}$ of the myosin arm 24 extended by the rotation of the myosin head 22 to the variable $L_{IR}$.

The second calculator 15b executes post-processing (S306) and stores a processing result in the internal memory. The process then returns. For example, the second calculator 15b sets a sum ($SumL_{IR}+L_{IR}$) of the value of the variable $SumL_{IP}$ and the value of the variable $L_{IR}$ to the variable $SumL_{IR}$ at S306. The second calculator 15b sets a sum ($SumXB_D+XB_D$) of the value of the variable $SumXB_D$ and the value of the variable $XB_D$ to the variable $SumXB_D$. The above-mentioned processing is performed by n step times in one step of the finite element analysis. Consequently, the number of consecutive times of the binding state of the myosin head 22 in one step of the finite element analysis is set to the variable $SumXB_D$. Furthermore, the sum of $L_{IR}$ in one step of the finite element analysis is set to the variable $SumL_{IR}$, and these sums are used for calculating an active stress in the finite element analysis, as will be described later.

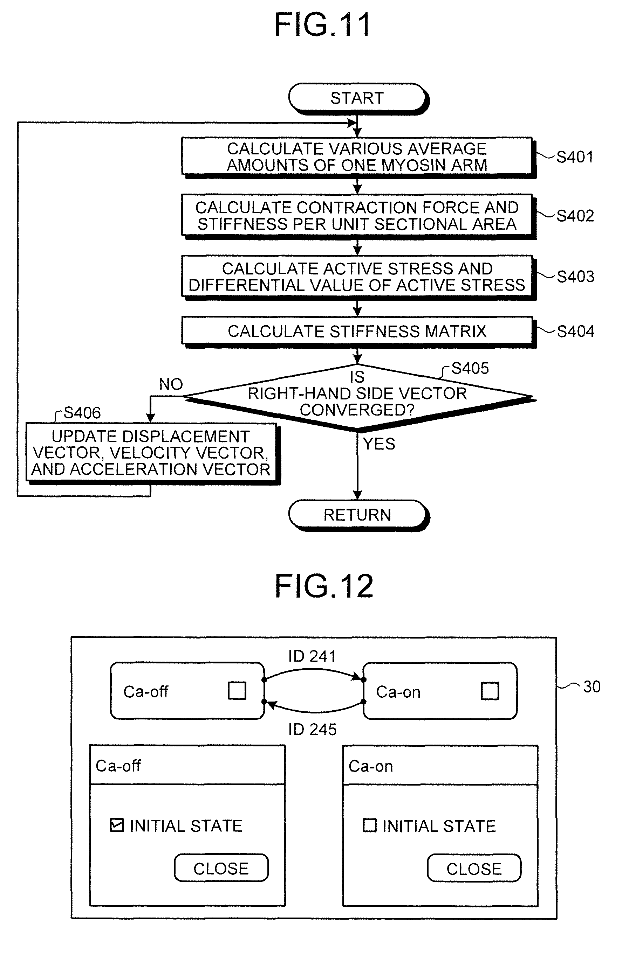

In FIG. 8, the second calculator 15b determines whether the value of the variable k is larger than n (S204). When the value of the variable k is not larger than n (No at S204), the second calculator 15b increments the value of the variable k by 1 (S205), and the process returns to S203. When the value of the variable k is larger than n (Yes at S204), the myocardial model processor 14a executes the finite element analysis (S206). FIG. 11 is a flowchart illustrating the procedures of the finite element analysis in the embodiment. As illustrated in FIG. 11, the myocardial model processor 14a calculates various average amounts of one myosin arm (S401). For example, the myocardial model processor 14a calculates an average value $L_{AVR}$ of stretch of the myosin arms 24 in one step of the finite element analysis at S401, using the following equation (7).

$$ L_{AVR} \;=\; \frac{\Delta t}{\Delta T}\left( XB_{D0}L_S(T) \;+\; SumL_{IR} \;+\; \Delta t\,\frac{SL_0}{2}\,SumXB_D\,\dot{\lambda}\!\left(T \;+\; \Delta T\right)\right) \qquad (7) $$

The myocardial model processor 14a calculates an average value $F_{MH}$ of forces of the myosin arms 24 and an average value $K_{MH}$ of stiffnesses of the myosin arms 24 in one step of the finite element analysis at S401, using the following equation (8).

$$ F_{MH} \;=\; \frac{dW_{arm}}{dL}\left(L_{AVR}\right),\; K_{MH} \;=\; \frac{d^2 W_{arm}}{dL^2}\left(L_{AVR}\right) \qquad (8) $$

Note that $W_{arm}$ is the elastic energy of a spring that is given as a function of the stretch L of the myosin arm 24.

The following equation (9) expresses virtual work $\delta W_{MH}$ of the myosin arm for a given variation $\delta\lambda$ of the stretch along the fiber direction.

$$ \delta W_{MH} \;=\; F_{MH}\,\frac{SL_0}{2}\,\delta\lambda \qquad (9) $$

The myocardial model processor 14a calculates an active stress $S_{active}$ such that virtual work made by the myocardial models 13a per unit volume and virtual work made by a group of myosin arms in one step of the finite element analysis are equal to each other for any variation $\delta E$ of strain on the myocardial continuum model, based on the following equation (10). The following describes an example of the method.

$$ \delta W_{active} \;=\; \frac{R_{SA}}{SA_0\left(SL_0 \,/\, 2\right)ns} \sum_{is=1}^{ns} \sum_{im=1}^{nm} \delta W_{MH}\left(im,\, is\right) $$
$$ =\; \frac{R_{SA}}{SA_0\,ns} \sum_{is=1}^{ns} \sum_{im=1}^{nm} F_{MH}\left(im,\, is\right)\,\delta\lambda \;=\; S_{active} \,:\, \delta E \qquad (10) $$

In the equation (10), "im" indicates the index of the myosin heads 22 on the myosin filament 23, and "is" indicates the index of the sarcomere models 13b in the element 13a_1. In addition, $R_{SA}$ indicates a proportion of the sarcomeres in the muscle, and $SA_0$ indicates a sectional area of one sarcomere model 13b. $S_{active}$ indicates an active stress tensor of the continuum model, $\delta E$ indicates a variation of a Green-Lagrange strain tensor, and a product of the two tensors indicates virtual work of the continuum.

The myocardial model processor 14a calculates a contraction force F of the myocardial model 13a per unit sectional area (S402), using the following equation (11) based on the equation (10).

$$F = \frac{R_{SA}}{SA_0 \, ns} \sum_{is=1}^{ns} \sum_{im=1}^{nm} F_{MH}\left(im, is\right) \tag{11}$$

The myocardial model processor 14a calculates a stiffness K of the myocardial model 13a per unit sectional area at S402, using the following equation (12).

$$K = \frac{\Delta t^2}{\Delta T} \frac{R_{SA}}{SA_0 \, ns} \frac{SL_0}{2} \sum_{is=1}^{ns} \sum_{im=1}^{nm} K_{MH}\left(im, is\right) \, SumXB_D\left(im, is\right) \tag{12}$$

The following equations (13) and (14) each indicate a relation between the variation of the Green-Lagrange strain tensor and the variation of the stretch $\lambda$ along the fiber direction. In consideration of the equation (13), it is known that the equation (10) is satisfied by the calculation of the active stress tensor $S_{active}$ as indicated by the following equation (15) using F calculated in the equation (11).

$$\delta\lambda = \frac{1}{\lambda} f \otimes f : \delta E \tag{13}$$

$$\delta\dot{\lambda} = -\frac{\dot{\lambda}}{\lambda^2} f \otimes f : \delta E + \frac{1}{\lambda} f \otimes f : \delta\dot{E} \tag{14}$$

$$S_{active} = \frac{F}{\lambda} f \otimes f \tag{15}$$

Thus, the myocardial model processor 14a calculates $S_{active}$ using the equation (15) (S403). When the finite element analysis is executed with an implicit method such as the Newmark-$\beta$ method, the myocardial model processor 14a calculates a stiffness matrix used in the implicit method (S404), using the following equations (16) and (17).

$$\frac{\partial S_{active}}{\partial E} = -\frac{F}{\lambda^2} f \otimes f \otimes \frac{\partial\lambda}{\partial E} + \frac{K}{\lambda} f \otimes f \otimes \frac{\partial\dot{\lambda}}{\partial E}$$
$$= -\frac{F + \dot{\lambda}K}{\lambda^3} f \otimes f \otimes f \otimes f \tag{16}$$

$$\frac{\partial S_{active}}{\partial \dot{E}} = \frac{K}{\lambda} f \otimes f \otimes \frac{\partial\dot{\lambda}}{\partial \dot{E}} = \frac{K}{\lambda^2} f \otimes f \otimes f \otimes f \tag{17}$$

These equations are derived from the relational equations (13) and (14) for the stretch $\lambda$ along the fiber direction and the temporal differentiation thereof and the Green-Lagrange strain tensor E and the temporal differentiation thereof. The myocardial model processor 14a calculates an equivalent nodal force from the active stress $S_{active}$ calculated by the equation (10) and the stiffness matrix based on the equation (16) and the equation (17), and superimposes them for all the elements to create a right-hand side vector and a coefficient matrix of the linear equation on the Newton-Raphson iteration at S404. When the right-hand side vector corresponding to a residual vector is sufficiently small (Yes at S405), the Newton-Raphson iteration is finished, a processing result is stored in the internal memory, and the process returns. When the right-hand side vector corresponding to the residual vector is not sufficiently small (No at S405), the myocardial model processor 14a solves the linear equation and updates a displacement vector, a velocity vector, and an acceleration vector of the continuum model based on the obtained solution. The process then returns to S401 and shifts to subsequent Newton-Raphson iteration. Note that the initial values of these respective vectors at the time step $T+\Delta T$ are assumed to be values of the vectors at the time T. As in the equation (7), the average stretch $L_{AVR}$ of each myosin arm in the time interval $[T, T+\Delta T]$ is calculated from the temporal differentiation of the stretch $\lambda$ along the fiber direction at the time $T+\Delta T$. That is to say, the motion of the continuum model is fed back to the calculation of the contraction force in a strongly coupled manner, so that an influence of the cross-bridge interactions in the time interval $[T, T+\Delta T]$ is appropriately reflected to the stiffness matrix. This can provide a stable calculation.

When the Newton-Raphson iteration is converged, the finite element analysis is finished. Turning back to FIG. 8, the myocardial model processor 14a updates the variable Ls for calculation at the next time step (S207), using the following equation (18). This update correctly resets, to Ls, the contribution of the shortening velocity in the stretch L of the myosin arm spring by the time $T+\Delta T$.

$$L_S(T + \Delta T) := L_{S0} - \Delta t X B_D \dot{u}(T + \Delta T) \qquad (18)$$

The receiver 14d receives the definition of the states and the transitions of the T/T units 20 and the definition of the states and the transitions of the myosin head 22 that are defined in the sarcomere models 13b. The receiver 14d then reflects the received contents to each model. FIG. 12 to FIG. 18 are views for explaining pieces of information that are received by the receiver in the embodiment. As illustrated in FIG. 12, when a user, such as a researcher studying the heart medically, inputs an instruction to display a screen on which the state and the transition of the T/T unit 20 are received through the input unit 11, the receiver 14d executes the following processing. Specifically, the receiver 14d causes the display unit 12 to display a screen 30 on which the state and the transition of the T/T unit 20 are received. The example of FIG. 12 illustrates the case where the two states of "Ca-off" and "Ca-on" are defined through the screen 30. Checking a check box next to "Initial State" sets the state corresponding to the checked check box to a state at the start time of the biological simulation processing. The example of FIG. 12 indicates that the state at the start time of the biological simulation processing is "Ca-off".

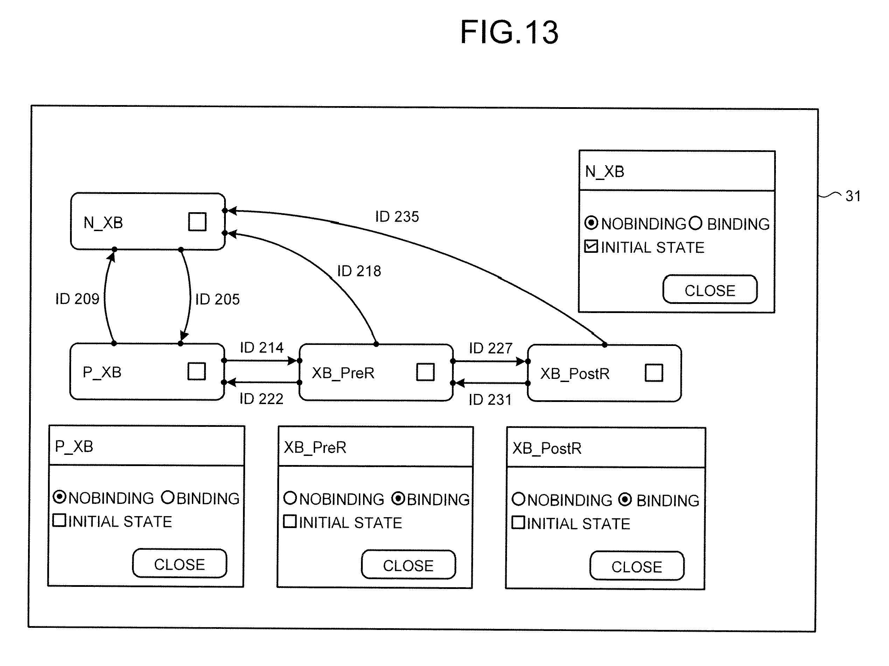

As illustrated in FIG. 13, when the user inputs an instruction to display a screen on which the state and the transition of the myosin head 22 are received through the input unit 11, the receiver 14d executes the following processing. Specifically, the receiver 14d causes the display unit 12 to display a screen 31 on which the state and the transition of the myosin head 22 are received. The example of FIG. 13 illustrates the case where four states of "N_XB", "P_XB", "XB_PreR" and "XB-PostR" are defined through the screen 31. Checking a check box next to "Initial State" sets a state corresponding to the checked check box to a state at the start time of the biological simulation processing. The example of FIG. 13 indicates that the state at the start time of the biological simulation processing is "N_XB". The screen 31 illustrated in FIG. 13 includes radio buttons for setting the respective four states to be "Nobinding (non-binding state)" or "Binding (binding state)". The example of FIG. 13 indicates that "N_XB" and "P_XB" are the non-binding state and "XB_PreR" and "XB_PostR" are the binding state.

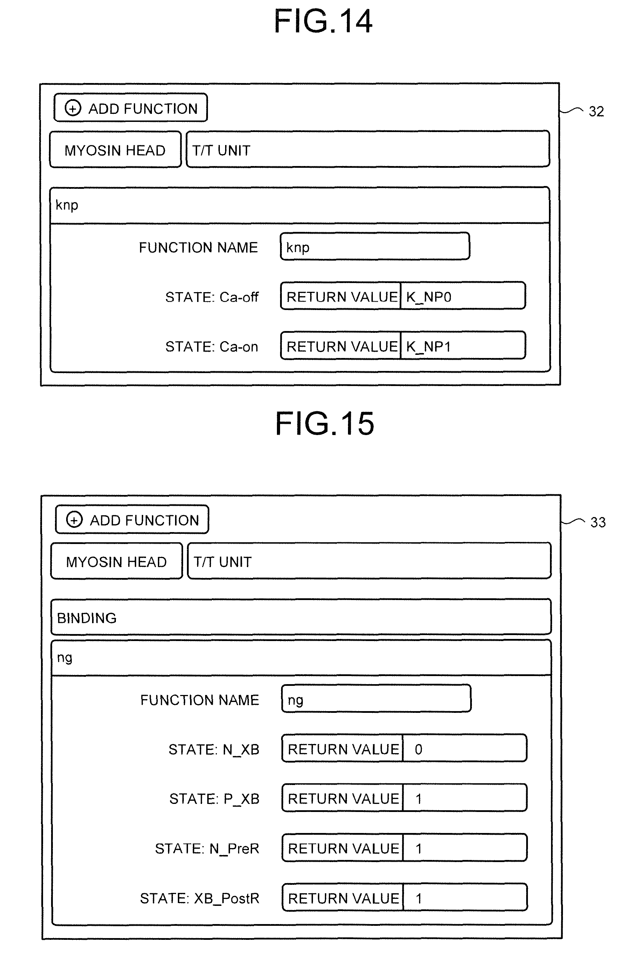

As illustrated in the examples of FIG. 14 and FIG. 15, when the user inputs an instruction to display a screen for defining a function through the input unit 11, the receiver 14d executes the following processing. Specifically, the receiver 14d causes the display unit 12 to display a screen 32 and a screen 33 for defining a function. The example of FIG. 14 indicates the case where a state function knp of the T/T unit 20 is defined through the screen 32. With the state function knp, for example, "K_NP0" is returned when the state is "Ca-off", and "K_NP1" is returned when the state is "Ca-on". The example of FIG. 15 indicates the case where a state function ng of the myosin head 22 is defined through the screen 33. With the state function ng, for example, "0" is returned when the state is "N_XB", and "1" is returned when the state is "P_XB", "XB_PreR", or "XB_PostR". The defined state function is referred to in the definition of the transition rates.

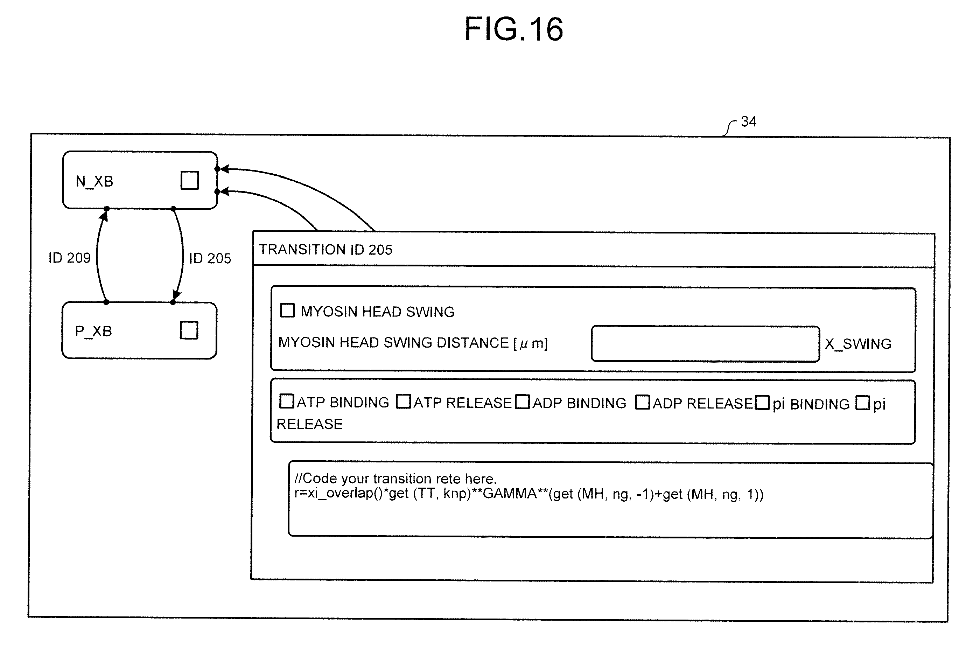

As illustrated in FIG. 16, when the user operates the input unit 11 to select an arrow from "N_XB" toward "P_XB", the receiver 14d causes the display unit 12 to display a screen 34 for defining a transition rate from "N_XB" to "P_XB". The example of FIG. 16 illustrates the case where the function knp returning a value in accordance with the state of the T/T unit 20 above the actin section just above the myosin head 22 with get(TT,knp) is used to define the transition rate from "N_XB" toward "P_XB". Furthermore, the example of FIG. 16 illustrates the case where the function values ng on the right and left myosin heads 22 with respect to the myosin head 22 with get(MH,ng, -1) and get(MH,ng,1) and a parameter larger than 1 is employed as GAMMA. Thus, cooperativity between the neighbor myosin heads 22 is expressed. In addition, xi_overlap() is a function to which the overlap state of the actin filaments 21 defined from the sarcomere length is reflected, and is a function obtained by multiplying the function $\chi_{RA}$ and the function $\chi_{LA}$ illustrated in FIG. 6.

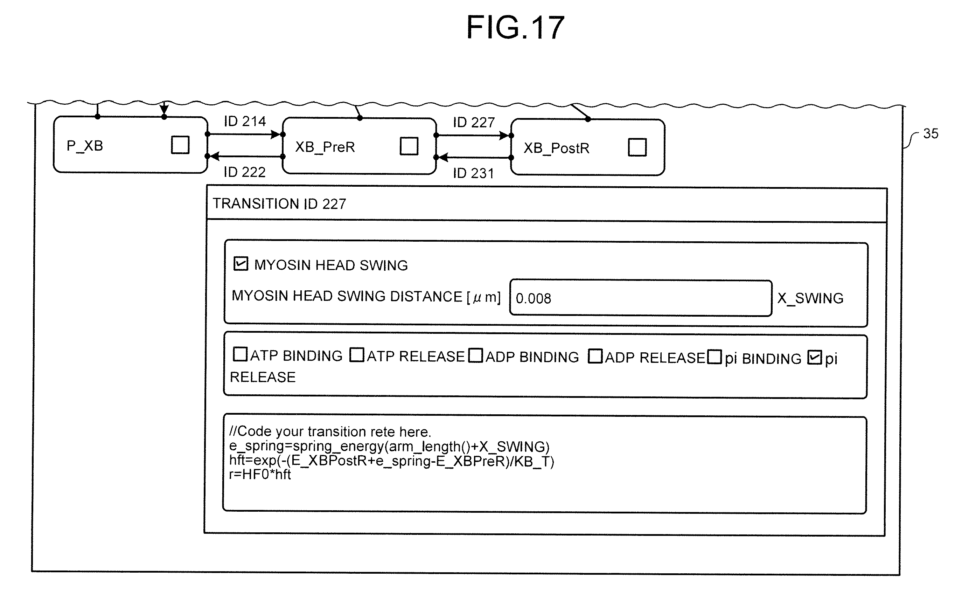

Furthermore, as illustrated in FIG. 17, when the user operates the input unit 11 to select an arrow from "XB_PreR" toward "XB_PostR", the receiver 14d performs the following processing. Specifically, the receiver 14d causes the display unit 12 to display a screen 35 for defining a transition rate from "XB_PreR" toward "XB_PostR" that involves the rotation of the myosin head 22. In the example of FIG. 17, when the transition is a transition involving the rotation of the myosin head 22, the user checks a check box next to "Myosin head swing". Furthermore, in the example of FIG. 17, the user inputs an increment of the stretch of the myosin arm 24 due to the rotation as X_SWING. In defining the transition rate, the stretch L of the myosin arm 24 in the original state "XB_PreR" of the myosin head 22 is called with the function arm_length(). The elastic energy of the myosin arm 24 with the stretch of the myosin arm, arm_length()+X_SWING, in the state after the transition is obtained with calling spring_energy. A transition rate r is defined with a Boltzmann factor defined from a total energy obtained by summing the elastic energy and a chemical energy E_XBPostR in the myosin head 22 after transition.

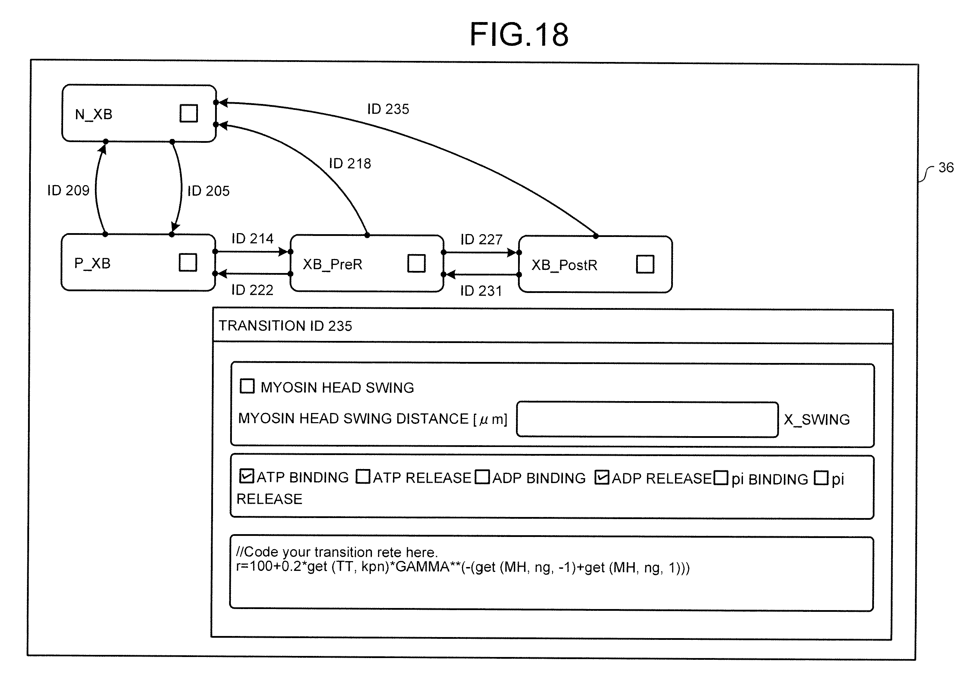

As illustrated in FIG. 18, when the user operates the input unit 11 to select an arrow from "XB_PostR" toward "N_XB", the receiver 14d performs the following processing. Specifically, the receiver 14d causes the display unit 12 to display a screen 36 for defining a transition rate from "XB_PostR" toward "N_XB" that involves dissociation of the myosin head 22. FIG. 18 illustrates the case where the user checks check boxes of the "ATP binding" and the "ADP release" in an assumption that ADP dissociates from the myosin head 22 and ATP binds thereto newly for the transition from the binding state to the non-binding state. The biological simulation device 10 according to the embodiment can calculate a consumption amount and a generation amount of molecules in the execution of the biological simulation, based on the above-mentioned specification by the user.

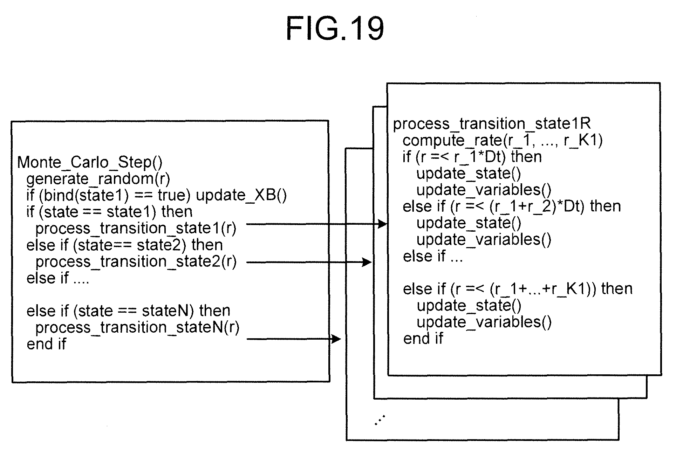

Furthermore, the receiver 14d automatically generates a code for executing the Monte Carlo simulation based on the states of the T/T units and the myosin head and transition information between the states that are defined through the above-mentioned input. FIG. 19 is a view illustrating a result of automatic generation of the code (Monte Carlo code) for executing the Monte Carlo step based on the definition of the states of the myosin head and the transitions between the states. As illustrated in FIG. 19, the code firstly generates a random number r in [0, 1], and executes processing in accordance with the current state. If the current state is the binding state, the variable ($XB_D$ and L) of the myosin head 22 is updated with the processing update_XB() as described in step S302 above. Next, the code executes one of process_transition_state to process_transition_stateN in accordance with the current state among N states. In this processing, the state is updated with update_stater() in accordance with the value of the random number r, and the variables are updated with update_variables() in accordance with the kind of transition, as described in step S305 above.

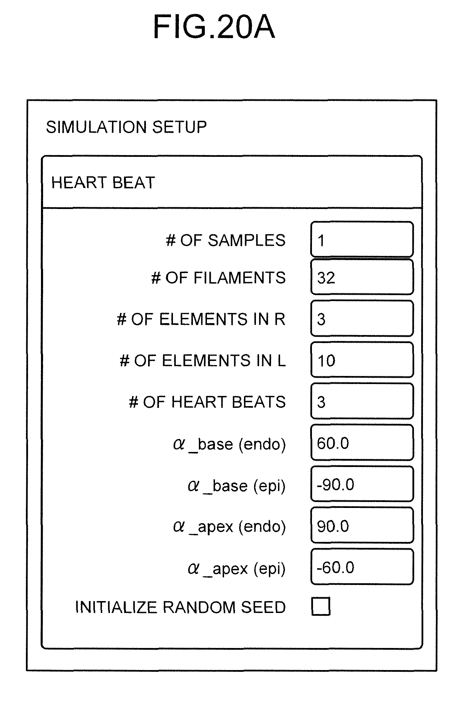

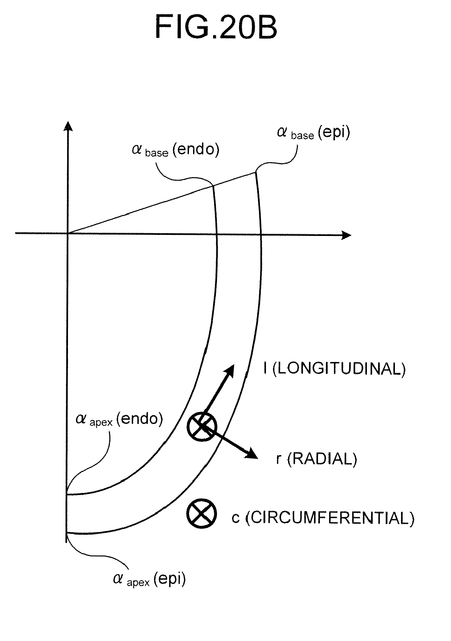

FIG. 20A and FIG. 20B illustrate examples where the code illustrated in FIG. 19 and the finite element code of the myocardial model 13a are combined as in the processing illustrated in FIG. 8 to execute simulation of heart beat through a coupling analysis with the myocardial model 13a. The examples employ a rotationally symmetric continuum obtained by rotating the section illustrated in FIG. 20B as the left ventricle model. FIG. 20A illustrates the case where the user specifies the number of elements in the wall penetrating direction and the lengthwise direction with "# of elements in R" and "# of elements in L" through the screen displayed on the display unit 12 by the receiver 14d. FIG. 20A illustrates the case where the user specifies the number of beats to be simulated with the "# of heart beats". FIG. 20A illustrates the case where the user specifies the distribution in the fiber direction, as illustrated in FIG. 20B, with "α_base" and "α_apex".

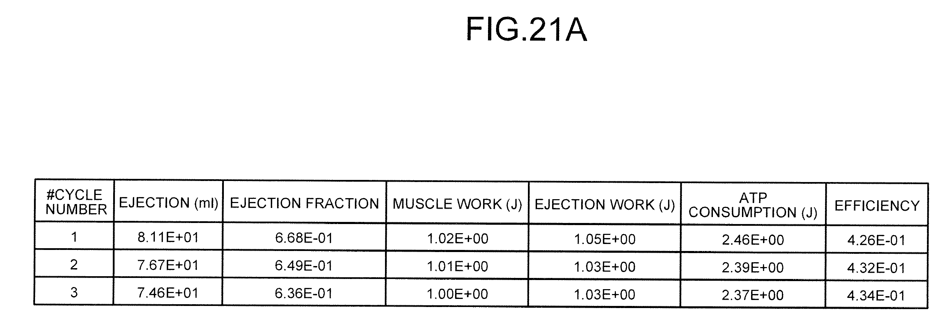

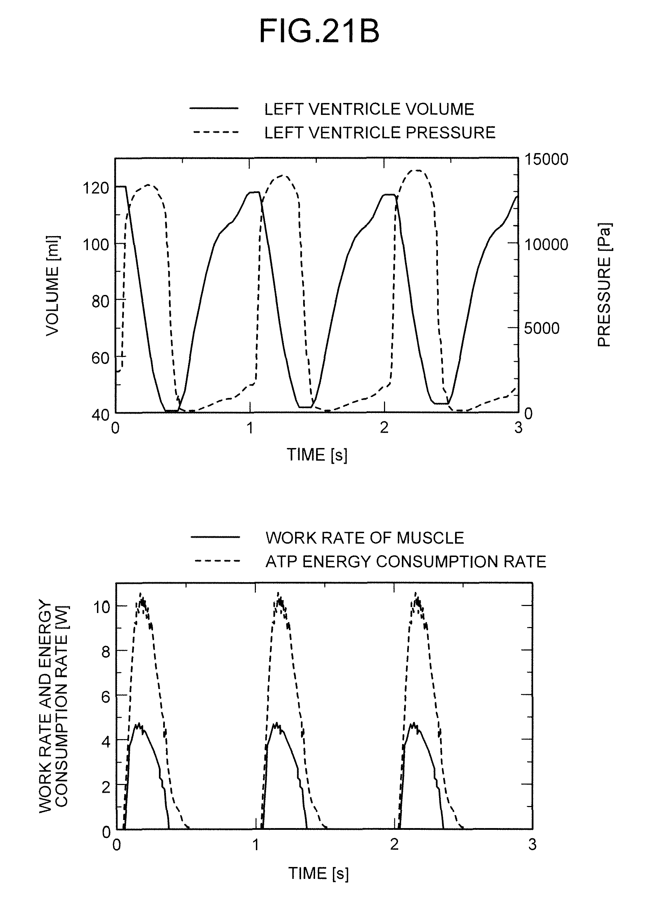

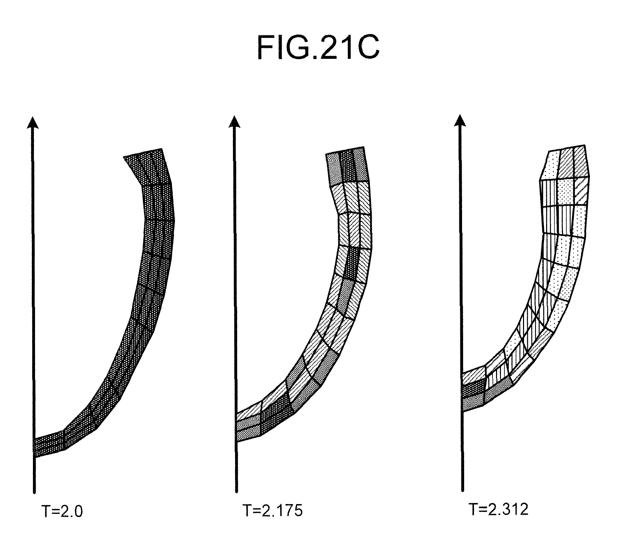

FIG. 21A to FIG. 21C are views illustrating examples of a simulation result. The simulation result illustrated in FIG. 21A includes output of an ejection amount (Ejection) by each beat, a ratio (Ejection Fraction) of the ejection amount and the energy, and a work amount (Muscle Work) made by the myocardial model. The simulation result illustrated in FIG. 21A also includes output of a work amount (Ejection work), an ATP consumption amount (ATP consumption), and efficiency (Efficiency) in the ejection of blood. The simulation result illustrated in FIG. 21B includes output of a temporal changes of the intraventricular volume and the intraventricular pressure, and temporal changes of conversion results of the work rate and ATP consumption rate, required for the ejection of blood, into hydrolysis energy. Furthermore, the simulation result as illustrated in FIG. 21C indicates temporal changes of integrated values of work made by the respective elements in one beat. Based on the simulation results as illustrated in FIG. 21A to FIG. 21C, the biological simulation device 10 according to the embodiment can analyze the relation between phenomena at the molecular level in the sarcomere models 13b and the performance of the heart beat.

As described above, the biological simulation device 10 according to the embodiment performs the following processing using the sarcomere model 13b that defines a plurality of states of a plurality of actins and a plurality of myosins in the sarcomere contained in the muscle of the biological body and transition rates among a plurality of states.

Specifically, the biological simulation device 10 calculates the states of the respective actins and the states of the respective myosins. The biological simulation device 10 calculates the behaviors of the respective actins and the behaviors of the respective myosins based on the respective states of the actins and the respective states of the myosins. Thus, the biological simulation device 10 can calculate the stochastic behaviors of the respective actins and myosins. This enables the biological simulation device 10 to provide an accurate simulation result.

Furthermore, the biological simulation device 10 according to the embodiment calculates the behavior of the sarcomere based on the behaviors of the actins and the behaviors of the myosins, and calculates the behavior of the muscle based on the behaviors of the respective sarcomeres. With this, the biological simulation device 10 according to the embodiment can couple the simulation of calculating the behavior of the muscle and the simulation of calculating the behaviors of the sarcomeres.

The biological simulation device 10 according to the embodiment calculates the behaviors of the respective actins and the behaviors of the respective myosins at the intervals $\Delta t$. The biological simulation device 10 then calculates the behavior of the muscle based on the behaviors of the respective sarcomeres that are calculated in $\Delta T$ at intervals $\Delta T$ longer than $\Delta t$. Thus, the biological simulation device 10 according to the embodiment calculates the behavior of the muscle using the behaviors of the respective actins and the behaviors of the respective myosins calculated by a plurality of times in $\Delta T$. Thus, the biological simulation device 10 can select the time interval $\Delta T$, at which the behavior of the muscle is calculated, in accordance with the convenience of the finite element analysis and can execute the phenomenon at the order of seconds, such as the heart beat, at an appropriate time. Even with such a gap of the time intervals, a coupling analysis with high accuracy can be performed because work amounts on the sarcomere models and the myocardial model are matched as illustrated in the equation (10). Furthermore, as indicated by the equation (7), the average stretch $L_{AVR}$ of the myosin arm in the time interval $[T,T+\Delta T]$ is calculated from the stretch velocity at the time $T+\Delta T$. This strongly couples the work amounts of the sarcomere models and the myocardial model thereby enabling a stable analysis in the time step $\Delta T$ having an appropriate length. This enables the simulation device 10 according to the embodiment to provide an accurate simulation result within a permissible calculation time.

The sarcomere model 13b, on which the biological simulation device 10 according to the embodiment performs processing, is defined such that when a myosin binding to an actin increases a probability that another myosin in a particular range from the myosin binds to the actin. Furthermore, the state transitions of each myosin head are defined in accordance with the overlap state of the actin filaments just above the myosin head. That is to say, the sarcomere model 13b is defined so as to indicate a behavior similar to that of a real sarcomere. The simulation device 10 calculates the states of the respective actins and the states of the respective myosins, using the sarcomere model 13b. As a result, the simulation device 10 can calculate the state similar to that of the real sarcomere.

The biological simulation device 10 calculates the length of the stretch of the myosin based on the length of the stretch of the myosin arm due to the binding of the myosin to the actin, the length of the stretch of the myosin arm due to the rotation of the myosin head, and an integrated value of the shortening velocity of the myosin.

Furthermore, the biological simulation device 10 according to the embodiment receives a plurality of states of a plurality of actins, transitions between the states of the actins, a plurality of states of a plurality of myosins, and transitions between the states of the myosins, which are defined for the sarcomere model 13b. The biological simulation device 10 generates the Monte Carlo code for executing the Monte Carlo step based on the received contents. This enables even a user such as a researcher who studies hearts medically but is not good at programming to perform a simulation of any desired sarcomere model without performing programming.

Although the embodiment relating to the disclosed device have been described hereinbefore, the device of the invention may be executed in various different modes other than the above-mentioned embodiment.

The entire processing or a part of the processing described in the above-mentioned embodiment that is performed automatically may be performed manually. The entire processing or a part of the processing described in the above-mentioned embodiment that is performed manually can be performed automatically with a known method.

The processing at the steps in the processes described in the above-mentioned embodiment can be subdivided into small pieces or be compiled as appropriate depending on various loads or usage conditions. Alternatively, any of the steps can be omitted.

Furthermore, the order of the pieces of processing at the steps in the processes described in the above-mentioned embodiment can be changed depending on various loads or usage conditions.

The components of the devices in the drawings are illustrated conceptually in function and are not necessarily configured as illustrated in the drawings physically. That is to say, the specific state of separation and integration of the devices are not limited to those illustrated in the drawings. The whole or a part thereof can be separated or integrated functionally or physically based on any desired unit depending on various loads or usage conditions.

Biological Simulation Program

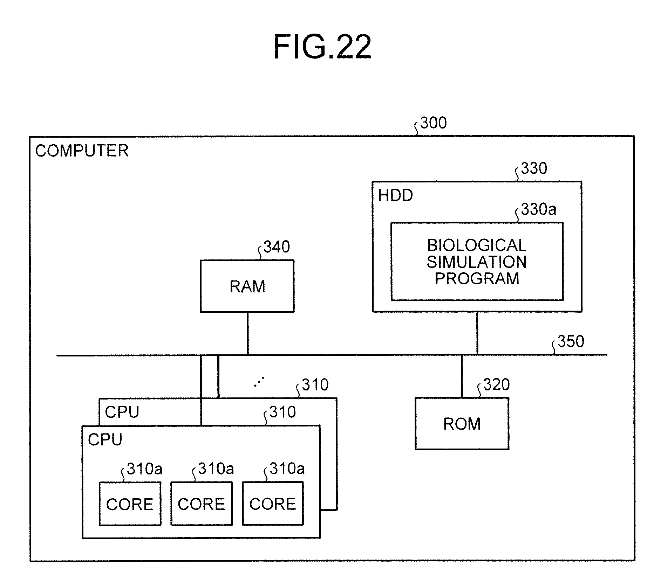

Various pieces of processing in the biological simulation device 10 described in the above-mentioned embodiment can be implemented through execution of a previously prepared program on a computer system such as a personal computer and a workstation. The following describes an example of a computer that executes a computer program having the same functions as those in the biological simulation device as described in the above-mentioned embodiment with reference to FIG. 22. FIG. 22 is a diagram illustrating the computer that executes a biological simulation program.

As illustrated in FIG. 22, a computer 300 includes a plurality of multi-core CPUs 310, a ROM 320, a hard disk drive (HDD) 330, and a RAM 340. Each CPU 310 includes cores 310a's. These components 310 to 340 are connected to one another via a bus 350.

The ROM 320 stores therein a basic program such as an OS. The HDD 330 previously stores therein a biological simulation program 330a exhibiting the same functions as those of the myocardial model processors 14a, the sarcomere model processors 14b, the first calculators 15a, and the second calculators 15b in the above-mentioned first embodiment. Note that the biological simulation program 330a may be separated as appropriate.

The CPUs 310 load the biological simulation program 330a from the HDD 330 and execute it.

The above-mentioned biological simulation program 330a is not necessarily stored in the HDD 330 initially.

For example, the computer 300 may load the biological simulation program 330a from the biological simulation program 330a stored in a "mobile physical medium" such as a flexible disk (FD), a compact disk read only memory (CD-ROM), a digital versatile disk (DVD), a magnetooptical disk, and an IC card that is inserted into the computer 300, and execute it.

Furthermore, the computer 300 may load the biological simulation program 330a from the biological simulation program 330a stored in "another computer (or server)" that is connected to the computer 300 through a public line, the Internet, a LAN, a wide area network (WAN), or the like, and execute it.

According to an aspect of an embodiment, an accurate coupling simulation result of molecular models having stochastic behaviors and motion of muscle as a continuum can be obtained.

## Claims

1. A biological simulation program causing a computer to execute:

   calculating states of a plurality of actins and states of a plurality of myosins in a sarcomere contained in a muscle of a biological body using a model that defines a plurality of states of the actins and a plurality of states of the myosins and transition rates between the states;
   calculating behaviors of the respective actins and behaviors of the respective myosins based on the states of the actins and the states of the myosins, respectively;
   calculating a behavior of the sarcomere based on the behaviors of the actins and the behaviors of the myosins; and
   calculating a behavior of the muscle based on the behavior of the sarcomere.

2. The biological simulation program according to claim 1, wherein
   at the calculating of the behaviors of the actins and the behaviors of the myosins, the behaviors of the actins and the behaviors of the myosins are calculated at an interval of a first time, and
   at the calculating of the behavior of the muscle, the behavior of the muscle is calculated at an interval of a second time longer than the first time based on behaviors of a plurality of sarcomeres calculated in the second time.

3. The biological simulation program according to claim 1 or 2, wherein at the calculating of the states of the actins and the states of the myosins, the states of the actins and the states of the myosins are calculated using the model that defines a myosin binding to an actin to increase a probability that another myosin in a particular range of the myosin binds to the actin.

4. The biological simulation program according to any one of claims 1 to 3, wherein at the calculating of the behaviors of the actins and the behaviors of the myosins, a length of stretch of one of the myosins is calculated based on a length of stretch of a myosin arm included in the myosin due to the binding of the myosin to the actin, a length of stretch of the myosin arm with rotation of a myosin head included in the myosin after the biding of the myosin to the actin, and an integrated value of shortening velocities of the myosin from the binding of the myosin to the actin to a current time.

5. The biological simulation program according to any one of claims 1 to 4, further causing the computer to execute:

   receiving the states of the actins and transitions between the states of the actins and the states of the myosins and transitions between the states of the myosins that are defined by the model.

6.  A biological simulation method of causing a computer to execute:

    calculating states of a plurality of actins and states of a plurality of myosins in a sarcomere contained in a muscle of a biological body using a model that defines a plurality of states of the actins and a plurality of states of the myosins and transition rates between the states;
    calculating behaviors of the respective actins and behaviors of the respective myosins based on the states of the actins and the states of the myosins, respectively;
    calculating a behavior of the sarcomere based on the behaviors of the actins and the behaviors of the myosins; and
    calculating a behavior of the muscle based on the behavior of the sarcomere.

7.  A biological simulation device comprising:

    a first calculator (14) configured to calculate states of a plurality of actins and states of a plurality of myosins in a sarcomere contained in a muscle of a biological body using a model that defines a plurality of states of the actins and a plurality of states of the myosins and transition rates between the states;
    a second calculator (14) configured to calculate behaviors of the respective actins and behaviors of the respective myosins based on the states of the actins and the states of the myosins, respectively; and
    a third calculator (14) configured to calculate a behavior of the sarcomere based on the behaviors of the actins and the behaviors of the myosins and calculate a behavior of the muscle based on the behavior of the sarcomere.

# FIG.1

BIOLOGICAL SIMULATION DEVICE ⌒10

CONTROLLER ⌒14

STORAGE UNIT ⌒13

⌒11
INPUT UNIT

⌒12
DISPLAY UNIT

MYOCARDIAL MODEL PROCESSOR

MYOCARDIAL MODEL PROCESSOR ⌒14a

⌒14a

MYOCARDIAL MODEL ⌒13a

SARCOMERE MODEL ⌒13b

SARCOMERE MODEL PROCESSOR

SARCOMERE MODEL PROCESSOR ⌒14b

⌒15a
FIRST CALCULATOR ⌒14b

⌒15b
SECOND CALCULATOR

⌒14c
DISPLAY CONTROLLER

⌒14d
RECEIVER

# FIG.2

# FIG.3

# FIG.4

$K_{on}[Ca^{2+}]$

$K_{off}$

Ca-off

Ca-on

FIG.5

# FIG.6

# FIG.7

```
        ┌─────────┐
        │  START  │
        └─────────┘
             │
             ▼
   ┌────────────────────────┐
   │ SIMULATION PROCESSING  │──── S101
   └────────────────────────┘
             │
             ▼
          S102
         ╱────────────╲
        ╱ IS TIME STEP ╲     NO      ┌─────────┐
       ╱   t FINAL      ╲──────────▶ │  t=t+1  │ ─── S103
        ╲ TIME STEP?   ╱             └─────────┘
         ╲────────────╱
             │ YES
             ▼
   ┌────────────────────────┐
   │  PERFORM CONTROL TO    │──── S104
   │ DISPLAY SIMULATION     │
   │       RESULT           │
   └────────────────────────┘
             │
             ▼
        ┌─────────┐
        │   END   │
        └─────────┘
```

# FIG.8

```
                    ┌──────────────┐
                    │    START     │
                    └──────┬───────┘
                           │
                           ▼
         ┌─────────────────────────────────────┐
         │   INITIALIZE AT TIME INTERVAL        │ ～S201
         │          [T, T+ Δ T]                 │
         └─────────────────┬───────────────────┘
                           │
                           ▼
         ┌─────────────────────────────────────┐
         │              k=1                     │ ～S202
         └─────────────────┬───────────────────┘
                           │
    ┌──────────────────────┤
    │                      ▼
    │    ┌─────────────────────────────────────┐
    │    │   MONTE CARLO SIMULATION            │ ～S203
    │    │          PROCESSING                 │
    │    └─────────────────┬───────────────────┘
    │                      │              ⌒S204
    │         NO           ▼
    │    ◄─────────◄    k>n?    ►
    │                      │
    │  ⌒S205               │ YES
    │  ┌──────────┐        ▼
    │  │  k=k+1   │   ┌─────────────────────────────┐
    └──┤          │   │  FINITE ELEMENT ANALYSIS    │ ～S206
       └──────────┘   └─────────────┬───────────────┘
                                    │
                                    ▼
                      ┌─────────────────────────────┐
                      │      POST-PROCESSING         │ ～S207
                      └─────────────┬───────────────┘
                                    │
                                    ▼
                            ┌──────────────┐
                            │   RETURN     │
                            └──────────────┘
```

FIG.9

# FIG.10

START

S301
IS
MYOSIN HEAD IN BINDING
STATE?
NO

YES

PRE-PROCESSING — S302

STATE CALCULATION — S303

S304
HAS STATE TRANSITIONED?
NO

YES

STATE TRANSITION PROCESSING — S305

POST-PROCESSING — S306

RETURN

# FIG.11

```
                    ┌─────────────┐
                    │    START    │
                    └─────────────┘
                           │
    ┌──────────────────────┼──────────────────────┐
    │                      ▼
    │   ┌──────────────────────────────────┐
    │   │  CALCULATE VARIOUS AVERAGE       │ ～S401
    │   │  AMOUNTS OF ONE MYOSIN ARM       │
    │   └──────────────────────────────────┘
    │                      │
    │                      ▼
    │   ┌──────────────────────────────────┐
    │   │  CALCULATE CONTRACTION FORCE AND │ ～S402
    │   │  STIFFNESS PER UNIT SECTIONAL AREA│
    │   └──────────────────────────────────┘
    │                      │
    │                      ▼
    │   ┌──────────────────────────────────┐
    │   │  CALCULATE ACTIVE STRESS AND     │ ～S403
    │   │  DIFFERENTIAL VALUE OF ACTIVE STRESS│
    │   └──────────────────────────────────┘
    │                      │
    │                      ▼
    │   ┌──────────────────────────────────┐
    │   │  CALCULATE STIFFNESS MATRIX      │ ～S404
    │   └──────────────────────────────────┘
    │                      │
    │                      ▼
    │             ╱─────────────────╲  ┌S405
    │       NO   ╱        IS          ╲
    │   ◄───────◄  RIGHT-HAND SIDE VECTOR ►
    │            ╲     CONVERGED?      ╱
    │  ┌S406      ╲─────────────────╱
    │   ▼                  │ YES
    │  ┌──────────────────────────┐      ▼
    │  │ UPDATE DISPLACEMENT      │  ┌─────────────┐
    │  │ VECTOR, VELOCITY VECTOR, │  │   RETURN    │
    │  │ AND ACCELERATION VECTOR  │  └─────────────┘
    │  └──────────────────────────┘
    └──────────┘
```

# FIG.12

```
┌──────────────────────────────────────────────────────────┐
│                         ID 241                            │
│   ┌──────────────────┐   ⤵      ┌──────────────────┐      │ ～30
│   │  Ca-off    ☐     │ ◄────►   │  Ca-on     ☐     │      │
│   └──────────────────┘          └──────────────────┘      │
│                         ID 245                            │
│                                                           │
│   ┌──────────────────┐          ┌──────────────────┐      │
│   │ Ca-off           │          │ Ca-on            │      │
│   ├──────────────────┤          ├──────────────────┤      │
│   │                  │          │                  │      │
│   │ ☑ INITIAL STATE  │          │ ☐ INITIAL STATE  │      │
│   │                  │          │                  │      │
│   │   ┌────────┐     │          │   ┌────────┐     │      │
│   │   │ CLOSE  │     │          │   │ CLOSE  │     │      │
│   │   └────────┘     │          │   └────────┘     │      │
│   └──────────────────┘          └──────────────────┘      │
└──────────────────────────────────────────────────────────┘
```

# FIG.13

EP 2 763 068 A2

~31

**N_XB**

⊙ NOBINDING ○ BINDING
☑ INITIAL STATE

[ CLOSE ]

N_XB □

ID 235

ID 218

ID 209    ID 205

P_XB □    ID 214    XB_PreR □    ID 227    XB_PostR □
          ID 222                ID 231

**P_XB**

⊙ NOBINDING ○ BINDING
□ INITIAL STATE

[ CLOSE ]

**XB_PreR**

○ NOBINDING ⊙ BINDING
□ INITIAL STATE

[ CLOSE ]

**XB_PostR**

○ NOBINDING ⊙ BINDING
□ INITIAL STATE

[ CLOSE ]

# FIG.14

```
┌─────────────────────────────────────────────────────────┐
│  ⊕ ADD FUNCTION                                          │  ～ 32
│  ┌──────────────┐ ┌──────────────────────────────────┐  │
│  │ MYOSIN HEAD  │ │ T/T UNIT                         │  │
│  └──────────────┘ └──────────────────────────────────┘  │
│  ┌───────────────────────────────────────────────────┐  │
│  │ knp                                               │  │
│  │  ┌─────────────────────────────────────────────┐  │  │
│  │  │   FUNCTION NAME   │ knp              │        │  │  │
│  │  │                                              │  │  │
│  │  │   STATE: Ca-off   │ RETURN VALUE │ K_NP0 │   │  │  │
│  │  │                                              │  │  │
│  │  │   STATE: Ca-on    │ RETURN VALUE │ K_NP1 │   │  │  │
│  │  └─────────────────────────────────────────────┘  │  │
│  └───────────────────────────────────────────────────┘  │
└─────────────────────────────────────────────────────────┘
```

# FIG.15

```
┌─────────────────────────────────────────────────────────┐
│  ⊕ ADD FUNCTION                                          │  ～ 33
│  ┌──────────────┐ ┌──────────────────────────────────┐  │
│  │ MYOSIN HEAD  │ │ T/T UNIT                         │  │
│  └──────────────┘ └──────────────────────────────────┘  │
│  ┌───────────────────────────────────────────────────┐  │
│  │ BINDING                                           │  │
│  └───────────────────────────────────────────────────┘  │
│  ┌───────────────────────────────────────────────────┐  │
│  │ ng                                                │  │
│  │     FUNCTION NAME    │ ng               │         │  │
│  │                                                   │  │
│  │     STATE: N_XB      │ RETURN VALUE │ 0 │         │  │
│  │                                                   │  │
│  │     STATE: P_XB      │ RETURN VALUE │ 1 │         │  │
│  │                                                   │  │
│  │     STATE: N_PreR    │ RETURN VALUE │ 1 │         │  │
│  │                                                   │  │
│  │     STATE: XB_PostR  │ RETURN VALUE │ 1 │         │  │
│  └───────────────────────────────────────────────────┘  │
└─────────────────────────────────────────────────────────┘
```

# FIG.16

EP 2 763 068 A2

# FIG.17

EP 2 763 068 A2

```
P_XB  ☐  — ID 214 →  XB_PreR  ☐  — ID 227 →  XB_PostR  ☐
         ← ID 222 —           ← ID 231 —
```

TRANSITION ID 227

☑ MYOSIN HEAD SWING

MYOSIN HEAD SWING DISTANCE [$\mu$m]  [ 0.008 ]  X_SWING

☐ ATP BINDING  ☐ ATP RELEASE  ☐ ADP BINDING  ☐ ADP RELEASE  ☐ pi BINDING  ☑ pi RELEASE

```
//Code your transition rete here.
e_spring=spring_energy(arm_length()+X_SWING)
hft=exp(-(E_XBPostR+e_spring-E_XBPreR)/KB_T)
r=HF0*hft
```

35

# FIG.18

TRANSITION ID 235

☐ MYOSIN HEAD SWING

MYOSIN HEAD SWING DISTANCE [$\mu$m]  [_____] X_SWING

☑ATP BINDING ☐ATP RELEASE ☐ADP BINDING ☑ADP RELEASE☐pi BINDING ☐pi RELEASE

//Code your transition rete here.
r=100+0.2*get (TT, kpn)*GAMMA**(-(get (MH, ng, -1)+get (MH, ng, 1)))

EP 2 763 068 A2

# FIG.19

```
Monte_Carlo_Step()
  generate_random(r)
  if (bind(state1) == true) update_XB()
  if (state == state1) then
    process_transition_state1(r)
  else if (state== state2) then
    process_transition_state2(r)
  else if ....

  else if (state == stateN) then
    process_transition_stateN(r)
  end if
```

```
process_transition_state1R
  compute_rate(r_1, ..., r_K1)
  if (r =< r_1*Dt) then
    update_state()
    update_variables()
  else if (r =< (r_1+r_2)*Dt) then
    update_state()
    update_variables()
  else if ...

  else if (r =< (r_1+...+r_K1)) then
    update_state()
    update_variables()
  end if
```

# FIG.20A

SIMULATION SETUP

HEART BEAT

| | |
|---|---|
| # OF SAMPLES | 1 |
| # OF FILAMENTS | 32 |
| # OF ELEMENTS IN R | 3 |
| # OF ELEMENTS IN L | 10 |
| # OF HEART BEATS | 3 |
| $\alpha$_base (endo) | 60.0 |
| $\alpha$_base (epi) | -90.0 |
| $\alpha$_apex (endo) | 90.0 |
| $\alpha$_apex (epi) | -60.0 |
| INITIALIZE RANDOM SEED | ☐ |

# FIG.20B

# FIG.21A

| #CYCLE NUMBER | EJECTION (ml) | EJECTION FRACTION | MUSCLE WORK (J) | EJECTION WORK (J) | ATP CONSUMPTION (J) | EFFICIENCY |
|---|---|---|---|---|---|---|
| 1 | 8.11E+01 | 6.68E-01 | 1.02E+00 | 1.05E+00 | 2.46E+00 | 4.26E-01 |
| 2 | 7.67E+01 | 6.49E-01 | 1.01E+00 | 1.03E+00 | 2.39E+00 | 4.32E-01 |
| 3 | 7.46E+01 | 6.36E-01 | 1.00E+00 | 1.03E+00 | 2.37E+00 | 4.34E-01 |

# FIG.21B

# FIG.21C

T=2.0                    T=2.175                    T=2.312

# FIG.22

COMPUTER ⌒300

HDD ⌒330

BIOLOGICAL SIMULATION PROGRAM ⌒330a

RAM ⌒340

⌒350

CPU ⌒310

CPU ⌒310

∴ ⌒310

CORE ⌒310a  CORE ⌒310a  CORE ⌒310a

ROM ⌒320

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PETERSON J.N. ; HUNTER W.C. ; BERMAN M.R.** Estimated time course of Ca2+bound to troponin C during relaxation in isolated cardiac muscle. *American Physiological Society,* 1991, H1013-H1024
- **HUNTER P.J. ; MCCULLOCH A.D. ; TER KEURS H.E.D.J.** Modeling the mechanical properties of cardiac muscle. *Progress in Biophysics & Molecular Biology,* 1998, vol. 69, 289-331
- **RAZUMOVA M.V. ; BUKATINA A.E. ; CAMPBELL K.B.** Stiffness-distortion sarcomere model for muscle simulation. *J. Appl. Physiol,* 1999, vol. 87, 1861-1876
- **RICE J.J. ; WANG F. ; BERS D.M. ; DE TOMBE P.P.** Approximate model of cooperative activation and crossbridge cycling in cardiac muscle using ordinary differential equations. *Biophys. J.,* 2008, vol. 95, 2368-2390